# DEMANDE DE BREVET EUROPEEN

(11) **EP 0 666 060 A1**
(43) Date de publication de la demande: **09.08.1995**
(21) Numéro de dépôt: 94110361.6
(22) Date de dépôt: 04.07.1994
(51) Int. Cl.: A61B 17/72

(54) **Vis aiguille**

(30) Priorité: 03.02.1994 FR 9401203
(71) Demandeur: SCIENCE ET MEDECINE (Société anonyme), F-75014 Paris (FR)
(72) Inventeur: Breard, Francis Henri, F-75014 Paris (FR); Audren, Jean-Louis, F-75017 Paris (FR)

(57) **Abrégé**

La présente invention concerne une vis aiguille pour l'ostéosynthèse des fractures de l'extrémité inférieure du radius, du péroné, ou de la clavicule comprenant :
- en l'une de ses extrémités, une broche mousse élastique destinée à être insérée dans le fût centro-médullaire,
- en son autre extrémité, une vis destinée à être fixée dans l'os épiphysaire.

## Description

### DOMAINE D'APPLICATION

La présente invention se rapporte à un mode d'ostéosynthèse spécifique aux fractures de l'extrémité inférieure du radius, de la malléole externe du péroné ou de la clavicule.

### ETATS DE LA TECHNIQUE ET PROBLEMES

Pour réunir les fragments osseux de ces fractures, il est connu d'utiliser à l'heure actuelle :
- soit des broches simples ou précourbées mises en place à foyer fermé, qui n'ont pas de tenue dans un os spongieux et risquent de migrer,
- soit des plaques vissées qui ont l'inconvénient de la voie d'abord à foyer ouvert, avec ses risques d'adhérence, de sepsie, et surtout le risque cutané dans les zones épiphysaires où il n'y a pas de tissu d'interposition entre le matériel et la peau.

### INVENTION

La présente invention se propose de remédier à ces inconvénients, et pour ce faire, elle a pour objet, une vis aiguille de type nouveau, caractérisée en ce qu'elle comprend en l'une de ses extrémités une vis destinée à être fixée dans l'os épiphysaire distal du radius, du péroné ou de la clavicule et en son autre extrémité une broche mousse et élastique destinée à être montée dans le fût centro-médullaire.

Avantageusement, la vis aiguille possède par sa broche mousse élastique, un rappel élastique permettant d'effectuer un couple mécanique entre la diaphyse et l'épiphyse de l'os fracturé.

A côté de cet avantage principal, la vis comprend en son extrémité supérieure une tête cylindrique.

On dispose ainsi d'une vis aiguille souple mise à foyer fermé qui par l'adjonction d'un pas de vis émoussé à l'une de ses extrémités permet d'obtenir une ostéosynthèse solide d'emblée par une prise appropriée dans l'os spongieux épiphysaire du radius, du péroné ou de la clavicule.

La rééducation du poignet, de la cheville ou de l'épaule immédiatement après l'intervention est alors possible, afin de prévenir la fonte musculaire et les rétractions articulaires.

Par ailleurs, elle est facilement extractible en cas de reprise (nécessite un simple dévissage, puis traction).

Finalement, contrairement à l'ostéosynthèse par plaque, l'implant n'est pas apparent sous la peau et la cicatrisation est considérablement réduite.

La réalisation de cette ostéosynthèse va être décrite plus en détail mais sans caractère limitatif en référence aux dessins annexés sur lesquels :
- la figure 1 représente une vis aiguille en vue de face,
- la figure 2 est un montage par vis aiguille d'une fracture du quart inférieur du radius,
- la figure 3 est un montage par vis aiguille d'une fracture de la malléole externe,
- la figure 4 est un montage par vis aiguille d'une fracture du quart externe de la clavicule;
La vis aiguille décrite par la figure 1 est réalisée de préférence en titane. Elle est monobloc et constituée :
- d'une tête cylindrique (1)
- d'une vis à filetage spongieux (2)
- d'une broche mousse élastique (3)
La broche mousse élastique est à bout sphérique (4) pour un usage centro-médullaire. Sa souplesse lui permet de suivre les incurvations anatomiques des cavités centro-médullaires. La vis (2) se fixe dans le spongieux épiphysaire de telle sorte que la vis aiguille ne peut migrer.

La tête cylindrique (1) est creusée d'un six pans femelle (5) permettant ainsi le vissage de la vis aiguille à l'aide d'un moteur ou d'un tournevis.

De plus, l'émoussage de la tête cylindrique la rend atraumatique pour les tendons passant à son voisinage, contrairement aux broches coupées à leur extrémité en fin de montage.

Le montage réalisé est alors beaucoup plus solide qu'avec des broches simples.

Plusieurs types de pas de vis sont bien entendu réalisés pour permettre de s'adapter aux différentes configurations anatomiques des épiphyses rencontrées et à la qualité de l'os épiphysaire rencontré (os jeune ou ostéoporotique).

### Fracture du quart inférieur du radius - Technique opératoire

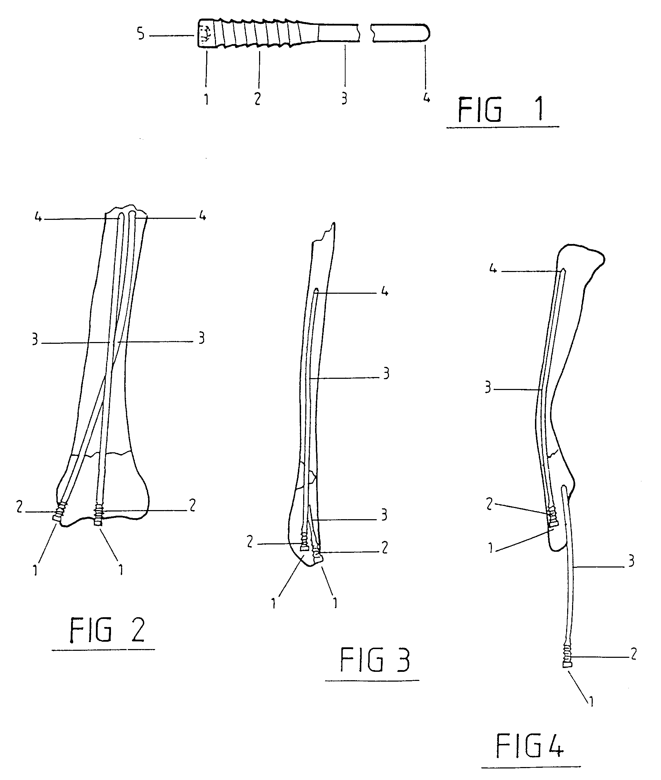

La figure 2 est un montage par vis aiguille d'une fracture du quart inférieur du radius. La fracture instable ostéosynthésée est réduite et stabilisée par deux vis aiguilles.

La réduction première de la fracture est faite par manoeuvres externes par la technique habituelle sous amplificateur de brillance.
- la voie d'abord est faite par deux petites incisions au bistouri en arrière, en dedans du tendon du deuxième radial et en dehors juste en arrière du tendon long extenseur du pouce, et en ayant soin de récliner la branche dorsale du nerf radial,
- une pointe carrée de 2mm permet de faire un pré-trou dans la corticale et dans le spongieux épiphysaire.

Cette trépanation est faite au ras de l'os sous chondral du radius qui est la seule zone réellement solide de l'épiphyse radiale.
- Les deux vis aiguilles sont sur un nez américain à la main montées dans la médullaire, elles se croisent et se courbent sur la corticale radiale opposée, une fois le foyer de fracture passé ; une fois montées, elles permettent le rappel élastique de la bascule postérieure et de la translation radiale de l'extrémité inférieure du radius.

Les vis aiguilles sont enfoncées à la main contre l'épiphyse au ras de la jonction entre la broche et la vis jusqu'à ce qu'elle bute sur l'épiphyse.

Les vis aiguilles sont alors vissées dans l'épiphyse radiale juste au ras de l'os sous-chondral qui assure un montage stable d'emblée.

Ce vissage effectué sous contrôle radio laisse juste dépasser la tête cylindrique qui ne gênera pas le glissement des tendons adjacents.

La peau est fermée à points séparés.

En fin d'intervention un simple pansement semi-compressif est mis en place, permettant la rééducation passive post-opératoire immédiate.

La mobilisation spontanée du poignet en post-opératoire est entreprise sans délai.

### Fracture de la malléole externe - Technique opératoire

La figure 3 est un montage par vis aiguille d'une fracture de la malléole externe.
- Voie d'abord externe dans le prolongement du péroné et sur la pointe de la malléole externe
Un décollement sous-cutané va permettre de mettre en place deux vis aiguilles,
- La trépanation de la malléole dans sa partie spongieuse se fait à la pointe carrée de 2mm.
- La réduction de la fracture est faite à l'aide de la pointe carrée pendant que l'on monte la première vis aiguille sous amplificateur de brillance.

Le passage des vis aiguilles au niveau du foyer de fracture et dans la médullaire du péroné se fait sous amplificateur de brillance.

L'extrémité mousse n'est pas dangereuse pour les éléments vasculo-nerveux et permet plusieurs tentatives de réduction jusqu'à ce que l'on trouve la médullaire avec sa résistance caractéristique.
- La mise en place de deux vis aiguilles va permettre de bloquer le foyer de fracture qu'il soit simple ou multifragmentaire du fait de l'encombrement des 2 broches de 20 mm.
- Le vissage du pas de vis dans le spongieux de la malléole externe complète le montage et le rend solide de première intention.

Seule la tête cylindrique des vis aiguilles est laissée affleurant la pointe de la malléole externe de telle sorte qu'il n'y a pas de conflit avec les tendons péroniers latéraux et la peau.

La peau est refermée à points séparés.

En cas de fracture isolée de la malléole péronière un simple bandage semi-compressif est mis en place permettant la rééducation passive post-opératoire immédiate.

### Fracture du quart externe de la clavicule - Technique opératoire

La figure 4 est un montage par vis aiguille d'une fracture du quart externe de la clavicule.

Voie d'abord externe en regard de la 2e courbure de la clavicule, juste en dedans de l'articulation acromio-claviculaire.
- Trépanation de la corticale supérieure du fragment externe en dedans de l'articulation acromio-claviculaire par une pointe carrée de 2mm ou bien à la mèche (deux trous).
- Réduction par manoeuvre externe et à l'aide de la pointe carrée qui guide le fragment externe pendant que l'on met en place la première vis aiguille qui passe le foyer de fracture.
- Les deux vis aiguilles sont montées en centro-médullaire, éventuellement précourbées au niveau de leur bout mousse, ce qui facilite le plus souvent la recherche de la médullaire du fragment interne.

L'encombrement centro-médullaire des deux vis aiguilles verrouille le foyer de fracture.
- Le vissage dans le spongieux du fragment externe complète le montage et évite toute migration des broches.

La peau est fermée à points séparés.
- Bandage simple de l'épaule, permettant la mobilisation immédiate de l'épaule.

## Revendications

1. Vis aiguille pour les fractures épiphysaires distales de l'extrémité inférieure du radius, du péroné ou de la clavicule, caractérisée en ce qu'elle comprend :
- en une de ses extrémités, une broche mousse élastique destinée à être insérée dans le fût centromédullaire,
- en son autre extrémité, une vis destinée à être fixée dans l'os épiphysaire.

2. Vis aiguille selon la revendication 1, caractérisée en ce que la broche mousse élastique possède une structure de rappel pour effectuer un couple mécanique entre la diaphyse et l'épiphyse de l'os fracturé.

3. Vis aiguille selon la revendicaion 1 ou 2, caractérisée en ce que la broche mousse élastique est à bout sphérique.

4. Vis aiguille selon la revendication 1 ou 2, caractérisée en ce que la vis comprend en son extrémité supérieure une tête cylindrique.

5. Vis aiguille selon une des revendications précédentes, caractérisée en ce que la tête cylindrique est creusée d'un multipans femelle permettant le vissage de la vis aiguille à l'aide d'un moteur ou d'un tournevis.

6. Vis aiguille selon une des revendications précédentes, caractérisée en ce qu'elle est en titane.
